# EUROPEAN PATENT APPLICATION

(11) **EP 3 789 365 A1**
(43) Date of publication of application: **10.03.2021**
(21) Application number: 18917254.7
(22) Date of filing: 16.08.2018
(51) Int. Cl.: C04B 38/06, A61L 9/014, A61L 9/16, C04B 35/581, C04B 35/584, C04B 35/636, B01J 20/30

(54) **POROUS CERAMIC HEATING ELEMENT COMPOSITION AND CERAMIC HEATING STRUCTURE MANUFACTURED OF SAME COMPOSITION**

(30) Priority: 02.05.2018 KR 20180050875
(71) Applicant: O&K Technology Co., Ltd., Asan-si, Chungcheongnam-do 31425 (KR)
(72) Inventor: KOH, Siyurn, Seoul 06294 (KR)
(74) Representative: BCKIP
(86) International application number: PCT/KR2018/009364
(87) International publication number: WO 2019/212094

(57) **Abstract**

The present invention relates to a porous ceramic heating element composition and a ceramic heating structure made of the composition, and the composition is formed by using: a mixture including a ceramic mixed powder, which is formed by mixing at least one of an aluminum nitride and a silicon nitride with a silicon carbide powder, and a silicon-based metal powder which is mixed with the ceramic mixed powder; a pore agent, wherein 0.5 parts by weight to 5 parts by weight of the pore agent is mixed with 100 parts by weight of the mixture; and a binder which is mixed with the mixture and maintains the bonding between the ceramic mixed powder and the metal powder, wherein 20 parts by weight to 30 parts by weight of the binder is included in 100 parts by weight of the mixture. The porous ceramic heating element composition according to the present invention has excellent thermal conductivity, corrosion resistance, and thermal shock resistance and, in particular, has many pores for adsorption and pyrolysis of external pollution substances, thereby enabling not only heating but also air purification capabilities.

## Description

### TECHNICAL FIELD

The present invention relates to a ceramic heating element composition which can be used in various fields such as a hot air blower, a dryer for a washing machine, a boiler, a food dryer, a hair dryer, an instant water heater, a hot and cold water dispenser, an animal washing and drying machine, an odor removing machine in the conventional industry and in the agriculture and stockbreeding field, a ventilation apparatus, a machine for removing components which are banned according to an environmental law and the like and, more specifically, to a porous ceramic heating element composition including a ceramic powder as a main material, and a ceramic heating structure made of composition.

### BACKGROUND ART

A ceramic heating element is a heating element with a property of generating heat by its resistance when power is applied. This ceramic heating element can be easily found in surroundings and is applied to, for example, a hair dryer, an instant water heater, a hot air blower, a dryer, a boiler and the like.

In addition, the ceramic heating element can be applied to an odor removing machine in the conventional industry and in the agriculture and stockbreeding field, a ventilation apparatus, and a machine for removing components which are banned according to an environmental law. Recently, researches and developments have been made so as to manufacture a deformation-free ceramic heating element, which has a reduced thermal loss, consumes less power, and has an improved durability, by improving the performance of the ceramic heating element.

For example, Korean Patent registration No. 10-1556938 discloses a feature of improving the performance of a heating element by securing the contact between the ceramic heating element and a metal terminal by inserting a metal mesh between the ceramic heating element and the metal terminal, which is fixed at an outskirt of the ceramic heating element.

However, the aforementioned conventional ceramic heating element has a drawback of having a high current density due to a small contact area between the ceramic heating element and the metal mesh. When the current density rises, heat is concentrated on the contact portion, which causes degradation and corrosion, and problems, such as the increase of the resistance value of the heating element with time, can occur.

In the meantime, a silicon carbide non-oxide ceramic heating element can be used as the element for a heater. However, the silicon carbide has a poor intrinsic water-resisting quality, and has a drawback that it easily loses the function as a heater due to degradation when it contacts moisture. In addition, the silicon carbide also has the problems that a separate control device needs to be essentially used together due to a large variation with temperature of the resistance value, and that, in particular, a transformer should be used together since its resistance is too low to be used as a heater.

### DETAILED DESCRIPTION OF THE INVENTION

### TECHNICAL PROBLEM

It is an objective of the present invention to provide a porous ceramic heating element composition which has excellent thermal conductivity, corrosion resistance, and thermal shock resistance and, in particular, has a plurality of pores for adsorption and pyrolysis of external pollution substances, thereby enabling not only heating but also air purification capabilities.

In addition, it is another objective of the present invention to provide a ceramic heating structure which has heating and air purification capabilities and effectively performs drying and sterilization activities when applied to various driers.

In addition, it is a still another objective of the present invention to provide a ceramic heating element composition which has low power consumption and has a proper resistance without the need for a transformer.

### TECHNICAL SOLUTION

In order to accomplish the objectives, a porous ceramic heating element composition according to the present invention can be formed by using:
a mixture including a ceramic mixed powder, which is formed by mixing at least one of an aluminum nitride and a silicon nitride with a silicon carbide powder, and a silicon-based metal powder which is mixed with the ceramic mixed powder;
a pore agent, wherein 0.5 parts by weight to 5 parts by weight of the pore agent is mixed with 100 parts by weight of the mixture; and
a binder which is mixed with the mixture and maintains the bonding between the ceramic mixed powder and the metal powder, wherein 20 parts by weight to 30 parts by weight of the binder is included in 100 parts by weight of the mixture.

In addition, the ceramic mixed powder included in the mixture amounts to 80 wt% to 95 wt% of the overall weight of the mixture.

In addition, a ratio of the aluminum nitride or the silicon nitride with respect to the silicon carbide powder can have a ranged between 0.1 parts by weight and 2 parts by weight in 100 parts by weight of the silicon carbide powder.

Also, the metal powder can include at least one element selected from an aluminum, a copper, and a nickel.

In addition, the pore agent is a carbon black or the graphite having a particle size between 0.1 *µ*m and 5 *µ*m, and the binder belongs to a methyl cellulose family.

In addition, in order to accomplish the objectives of the present invention, a ceramic heating structure according to the present invention is manufactured through molding by using:
a mixture including a ceramic mixed powder, which is formed by mixing at least one of an aluminum nitride and a silicon nitride with a silicon carbide powder, and a silicon-based metal powder which is mixed with the ceramic mixed powder;
a pore agent, wherein 0.5 parts by weight to 5 parts by weight of the pore agent is mixed with 100 parts by weight of the mixture; and
a binder which is mixed with the mixture and maintains the bonding between the ceramic mixed powder and the metal powder, wherein 20 parts by weight to 30 parts by weight of the binder is included in 100 parts by weight of the mixture, and the ceramic heating structure comprises a plurality of air paths through which the air to be heated passes.

Also, the heating structure includes pores for adsorbing and removing by pyrolysis a pollution substance in the air passing through the air path, wherein the pores are formed by removing the pore agent by the heat applied while manufacturing the ceramic heating structure.

### ADVANTAGEOUS EFFECTS

The porous ceramic heating element composition according to the present invention formed as above has excellent thermal conductivity, corrosion resistance, and thermal shock resistance and, in particular, has many pores for adsorption and pyrolysis of external pollution substances, thereby enabling not only heating but also air purification capabilities.

In addition, the ceramic heating structure according to the present invention has heating and air purification capabilities and effectively performs drying and sterilization activities when applied to various driers.

### DESCRIPTION OF THE DRAWINGS

FIG. 1 is a perspective view illustrating an embodiment of the ceramic heating structure according to an embodiment of the present invention.
FIG. 2 is a diagram for describing the operation of the ceramic heating structure according to an embodiment of the present invention.
FIG. 3 is an enlarged image taken from a porous ceramic heating element composition according to an embodiment of the present invention.
FIG. 4 is a front view illustrating another embodiment of the ceramic heating structure according to an embodiment of the present invention.

### BEST MODE

In the following, an embodiment of the present invention will be explained in detail by referring to the appended figures.

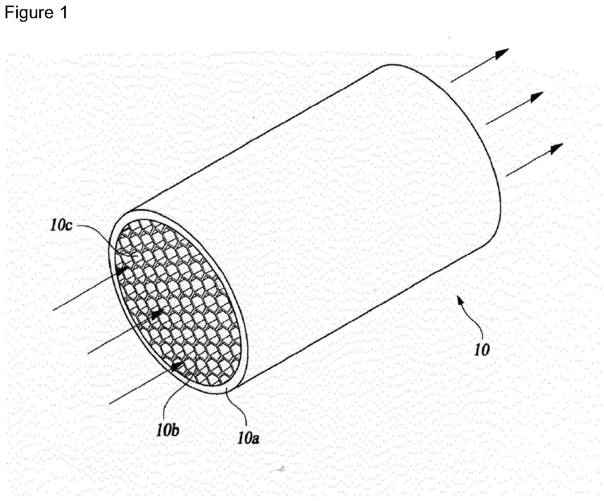

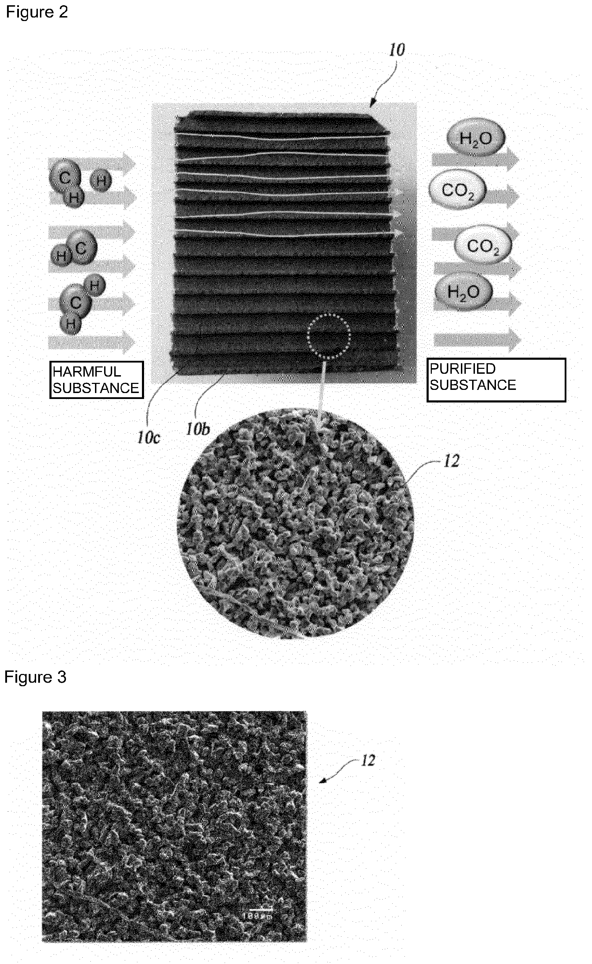

FIG. 1 is a perspective view illustrating an embodiment of the ceramic heating structure 10 according to an embodiment of the present invention. In addition, FIG. 2 is a diagram for describing the operation of the ceramic heating structure 10 and FIG. 3 is an enlarged image taken from a portion of the ceramic heating structure 10.

At first, the composition 12, which constitutes the ceramic heating structure 10, has a basic configuration including a mixture, a pore agent, and a binder.

The mixture includes a metal powder and a ceramic mixed powder and forms an overall structure of the heating structure 10. The ceramic mixed powder included in the mixture amounts to 80 wt% to 95 wt% of the overall weight of the mixture. That is, 5 wt% to 20 wt% of the mixture is the metal powder. The metal powder is related to the resistance value of the heating structure 10. As the content of the metal powder increases, the resistance value decreases, which causes the calorific value to decrease. The ceramic mixed powder is a powder made by mixing the silicon carbide (SiC) powder with an aluminum nitrate (AIN) or a silicon nitrate (Si₃N₄). The aluminum nitride and the silicon nitride can be used separately and they also can be used together. The mixing ratio of the aluminum nitrate or the silicon nitride with respect to the silicon carbide is 0.1 to 2 parts by weight in 100 parts by weight of the silicon carbide.

As known in the art, the aluminum nitride has an excellent thermal conductivity, which improves the efficiency of the heating element containing the aluminum nitride. In addition, the silicon carbide has properties such as excellent corrosion resistance, thermal shock resistance, thermal conductivity and the like, and the silicon nitride has good heat resistance, strength, thermal shock resistance and the like.

In addition, the metal powder includes a silicon-based metal powder or an aluminum powder, a copper powder, and a nickel powder. These powders can be used separately or more than two of the powders can also be selected and used. The metal powder is distributed inside the mixture and is an element for affecting the resistance value of the heating element. That is, the resistance value of the heating structure is adjusted by the content of the metal powder.

The metal powder is mixed with the ceramic mixed powder so as to make the heating structure 10 have a resistance between 10⁻¹ Ω and 10³ Ω such that the heating structure 10 can perform the functions of a heater.

An average particle diameter of the metal powder varies with the particle diameter of the ceramic mixed powder and, for example, when the particle diameter of the ceramic mixed powder lies between 1 *µ*m and 50 *µ*m, the average particle diameter of the metal powder can range from 50 nm to 300 nm.

When the average particle diameter of the ceramic mixed powder is smaller than 1 *µ*m, the ceramic mixed powder cannot be easily mixed with the metal powder while, when the average particle diameter is greater than 50 *µ*m, the resistance becomes excessively large and functionalities of a heater are degraded.

As for the metal powder, when the average particle diameter of the metallic power is smaller than 50 nm, the function of electrically connecting the ceramic mixed powder with the metallic power is restricted while, when the average particle diameter is greater than 300 nm, the metallic power cannot be easily mixed with the ceramic mixed powder. The metal powder and ceramic mixed powder are converted into a slurry phase by adding water, the pore agent and the binder into the powders and mixing the result for 5 to 15 hours, and the heating structure 10 is formed from the result through extrusion and heat treatment processes.

The pore agent is mixed at a predetermined ratio inside the mixture in which the metal powder and the ceramic mixed powder are mixed, and then is removed during a subsequent pyrolysis process, which enables a plurality of pores to remain inside the mixture. That is, the positions which have been occupied by the pore agent now turn into the pores.

The pore agent is a carbon-based powder and has a particle size between 0.1 *µ*m and 5 *µ*m, and the mixing ratio of the pore agent with respect to the mixture amounts to 0.5 parts by weight to 5 parts by weight in 100 parts by weight of the mixture. The content or the particle size of the pore agent can vary according to application objectives.

In addition, a carbon black or a graphite can be used as the pore agent. As known in the art, the carbon black or the graphite can be removed by the heat between 300°C and 600 °C. As a matter of fact, the carbon black or the graphite is used as the pore agent in the ceramic heating structure 10 according to the embodiment since the heat is generated in the aforementioned range during a manufacturing process through molding.

In the meantime, the binder is mixed with the mixture and functions to maintain the bonding between the ceramic mixed powder and metal powder. The mixing ratio of the binder amount to 20 parts by weight to 30 parts by weight in 100 parts by weight of the mixture. In particular, a methyl cellulose family (MC family) binder is used as the binder.

FIG. 1 is a perspective view of the ceramic heating structure 10 which is formed by mixing the ceramic mixed powder, the metal powder, the pore agent, and binder with one another to convert them into a slurry and then performing an extrusion molding at a high temperature and a high pressure. The shape of the ceramic heating structure 10 can be varied as necessary.

The ceramic heating structure 10 shown in FIG. 1 includes a housing portion 10a of a generally cylindrical shape, and a partition portion 10b of a honeycomb shape which is housed inside the housing portion 10a and provides a plurality of air paths 10c. The housing portion 10a and the partition portion 10b have been simultaneously formed by an extrusion molding, and therefore form one entity.

The air to be heated is introduced into one side air path 10c of the ceramic heating structure 10, moved and heated along the air path 10c, and then discharged in the opposite direction at a heated state.

In particular, as shown in FIG. 2, the harmful substance, which has been introduced into the ceramic heating structure 10, undergoes a purification process while passing through the air path 10c to be heated. That is, the polluted indoor air, which has been introduced into the heating structure 10, is subject to the purification process and discharged as a purified substance.

This purification process of the harmful substance is realized by the pores in the organization which constitutes the heating structure. That is, when the polluted air including the harmful substance passes through the air path 10c and is heated, the harmful substance is adsorbed by the pores inside the air path 10c, and the adsorbed harmful substance inside the pores is pyrolyzed by the heat generated inside the heating structure and by the far infrared radiation. For example, a hydrocarbon is pyrolyzed into a hydrogen, water, and a carbon dioxide, an acetaldehyde is pyrolyzed into a carbon dioxide and water, and a carbon monoxide is pyrolyzed into a carbon dioxide. In addition, fungi and bacteria in the air instantly become extinct.

As a result, the ceramic heating structure according to the present invention has the capability of removing the harmful substance included in the air through a pyrolysis process while heating the air which passes through the inner portion of the ceramic heating structure.

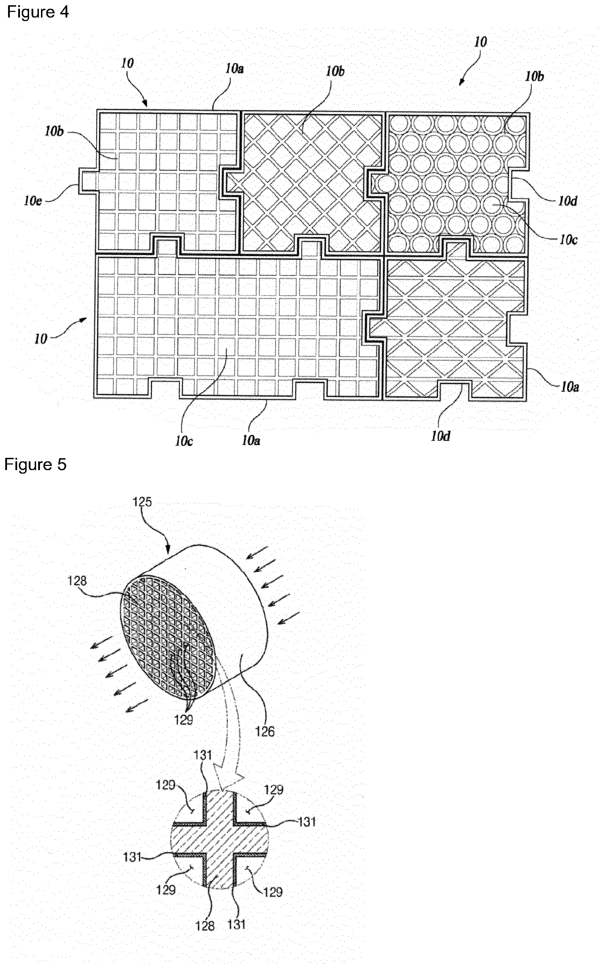

FIG. 4 is a front view illustrating another embodiment of the ceramic heating structure 10 according to an embodiment of the present invention. As shown in the figure, it is possible to manufacture the ceramic heating structure 10 in a generally rectangular shape and combine a plurality of ceramic heating structures with one another.

The ceramic heating structure 10 shown in FIG. 4 includes a housing portion 10, which has a generally rectangular cross-section shape and includes an insertion groove portion 10d and a protrusion portion 10e at a perimeter thereof, and a partition portion 10b which fills the inner portion of the housing portion 10 and provides an air path 10c. The partition portion 10b can have a grid-like pattern or a hollow tube-like pattern in addition to the honeycomb configuration.

The insertion groove portion 10d houses the protrusion portion 10e of a neighboring heating structure 10. By inserting the protrusion portion 10e into the insertion groove portion 10d as shown, the plurality of ceramic heating structures 10 come to form one entity.

### MODE OF THE INVENTION

The ceramic heating element composition according to another embodiment of the present invention includes a ceramic-and-metal mixed powder, a dispersion agent, and a binder.

The ceramic-and-metal mixed powder includes a non-oxide ceramic powder and a metal powder.

The non-oxide ceramic powder includes non-oxide ceramics. The non-oxide ceramics are formed by combining a carbide, a nitride, a boride ceramic and the like with a metal element (Si, Ti, Al, Zr and the like) and an element such as a carbon (C), a nitrogen (N), and a boron (B).

In this case, the non-oxide ceramic powder includes at least one of an aluminum oxide (AIN), a silicon carbide (SiC), and a silicon nitrate (Si₃N₄).

The aluminum oxide has a preferable thermal conductivity. Therefore, it is possible to manufacture a high efficiency heater when the heater is manufactured by using the aluminum oxide as a main material.

The silicon carbide has excellent properties of corrosion resistance, thermal shock resistance, heat conductivity and the like and, therefore, it is possible to manufacture a heater with high corrosion resistance when the heater is manufacture by using the silicon carbide.

The silicon nitride has excellent heat resistance, strength, thermal shock resistance and the like. When the heater is manufactured by using the silicon nitride, it is possible to manufacture a heater with a preferable durability.

According to the present invention, it is possible to use only the aluminum oxide as the non-oxide ceramic powder material. Alternatively, the silicon nitride may be used as the non-oxide ceramic powder material. Furthermore, it is also possible to mix the aluminum oxide with the silicon nitride to form the non-oxide ceramic powder.

The non-oxide ceramic powder has a resistance value around 10⁷ Ω, and the resistance is too high for the power to function as a heater.

Therefore, according to the present invention, a metal powder is mixed with the non-oxide ceramic powder. The metal powder is mixed with the non-oxide ceramic powder such that the ceramic heating element has a resistance between 10⁻¹ Ω and 10³ Ω and functions as a heater. In this case, the metal powder can include at least one element selected from an aluminum (Al), a copper (Cu), and a nickel (Ni).

It is preferred that the non-oxide ceramic powder according to the present invention includes at least one of an aluminum nitrate (AIN) and a silicon nitrate (Si₃N₄). The aluminum nitrate (AIN) and the silicon nitrate (Si₃N₄) have the advantages of high heat resistance, strength, and thermal shock resistance, and a small variation with temperature of the resistance, but they have poor electric conductivities. In order to compensate for this, the metal powder components are invited to be interposed between the non-oxide-based ceramic powder components. In this case, the electrical conductivity can be adjusted by properly adjusting the material of the metal powder.

In this case, it is preferred that the average particle diameter of the non-oxide ceramic powder lies between 1 *µ*m and 50 *µ*m, and it is preferred in this case that the average particle diameter of the metal powder lies between 50 nm and 300 nm. It is because, when the average particle diameter of the non-oxide ceramic powder is smaller than 1 *µ*m, the ceramic power is not mixed well with the metal powder while, when the average particle diameter of the non-oxide ceramic powder is greater than 50 *µ*m, the resistance becomes too large for the function of a heater. In addition, it is because, when the average particle diameter of the metal powder is smaller than 50 nm, it is not easy to connect non-oxides with each other to allow them to conduct electricity while, when the average particle diameter of the metal powder is greater than 300 nm, the metal powder cannot be mixed with the non-oxide ceramic powder so as to have an adequate overall conductivity.

It is possible to convert the metal powder and the ceramic mixed powder into a slurry phase by adding water and mixing the result for 5 to 15 hours.

In this case the, result further includes a dispersion agent and a binder.

The dispersion agent enables the non-oxide ceramic powder and the metal powder to be easily mixed with each other. At least one material in a fatty acid family with a polymer shape can be used as the dispersion agent. In this case, it is preferred that 3 to 30 parts by weight of the dispersion agent are included in 100 parts by weight of the ceramic-and-metal mixed powder.

The binder enables the metal powder and the non-oxide ceramic powder to bond together. A polyvinyl alcohol (PVA), a polyvinyl carbonate (PVC) and the like can be used as the binder. It is used so as to enable the metal powder components to be interposed between the non-oxide ceramic powder components and to allow the ceramic heating element to conduct electricity. In this case, it is preferred that 2 to 30 parts by weight of the binder are included in 100 parts by weight of the ceramic-and-metal mixed powder.

When describing the reactions made by the binder in more detail, (A) a PVA and a PVC react with a solvent, and undergo a decomposition and ionization processes to be converted into a free proton and an anionic binder (Hydroxyl group). (B) The free proton is adsorbed at a surface of the powder to convert it to a positively charged surface. (C) The anionic binder is attracted toward the positively charged powder surface by the Coulomb's force, and a non-polar tail is laid behind toward the solvent media.

This configuration allows a steric hindrance repulsion which enables the metal powders to be interposed between the non-oxide ceramic powders and evenly dispersed.

In the meantime, it is preferred that a methyl cellulose is further included as an additive. The methyl cellulose improves the molding performance of an extrusion molding process into a pipe shape having a plurality of paths.

In this case, it is preferred that 2 to 30 parts by weight of the methyl cellulose are included in 100 parts by weight of the ceramic-and-metal mixed powder. It is because, when less than 2 parts by weight of the methyl cellulose are included, the extrusion molding cannot be performed properly while, when more than 30 parts by weight of the methyl cellulose are included, it is difficult to maintain the shape at a process after molding.

In the meantime, the metal powder can further include a silicon.

In addition, the non-oxide ceramic powder can further include a silicon carbide (SiC). The silicon carbide has an excellent electrical conductivity. Therefore, when the silicon carbide is mixed with one of the aluminum oxide (AIN) and the silicon nitrate (Si₃N₄) having insulation properties, they can have an adequate electrical conductivity.

As shown in FIG. 5, when the slurry is extruded and dried, an external shape of the ceramic heating element 125 can be made, and the result is sintered and heat treated at a high temperature higher or equal to 1400°C so as to form the ceramic heating element 125. The heat treatment is a process in which the ceramic heating element 125 is heated to a temperature lower than the sintering temperature, and the hardness of the ceramic heating element 125 can be enhanced by the heat treatment.

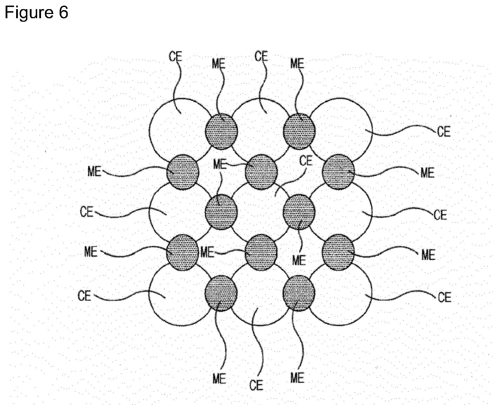

In this case, the metal powder (ME) components are interposed between the non-oxide ceramic powders (CE) as shown in FIG. 6, which allows the ceramic heating element 125 to conduct electricity. It is possible to change the resistance value of the ceramic heating element 125 by changing the weight % of the metal powder (ME) component. Since the ceramic heating element 125 is formed with an enlarged heat exchange surface through the extrusion molding and sintering, it becomes compacter than the heater made of a metallic material, which reduces an installation area and a manufacturing cost while improving the energy efficiency.

The ceramic heating element can be used effectively in various fields such as a hair dryer, a hot air blower, an instant water heater, a hot and cold water dispenser, a dryer for a washing machine, a gas stove, a boiler, an exhaust gas reducing device and the like.

In the meantime, although the outer wall 126 of the ceramic heating element 125 shown in FIG. 5 has a pipe-like shape with a circular cross-section, the shape of the cross-section is not restricted thereto, and the cross-section can have a polygonal pipe-like shape such as a triangle, a rectangle and the like.

As shown in an enlarged shape in FIG. 5, a catalyst 131 is applied on an outer surface of the partition inner wall 128. Although not shown in FIG. 1, the catalyst 131 can also be applied on an outer surface of the outer wall 126. The aqueous catalyst 131 can be sprayed on the ceramic heating element 125, or the catalyst 131 can be applied by dipping the ceramic heating element 125 in the aqueous catalyst 131 and then drying the result. For example, the catalyst 131 can be a platinum (Pt)-based catalyst or a palladium (Pd)-based catalyst.

The catalyst 131 promotes the decomposition which decomposes at least one component of the components included in the air in the inner space. In addition, for example, an incomplete combustion substance such as a carbon monoxide, a hydrocarbon and the like or a substance causing an odor can be discharged from an object to be dried, and the catalyst 131 can promote the decomposition of the incomplete combustion substance or the odor-causing substance. As a result, the drying quality of the object to be dried can be further enhanced.

As explained in the above, when the ceramic heating element having a plurality of paths is manufactured by the ceramic heating element composition according to the present invention, an excellent corrosion resistance can be obtained against an acid, an alkali, moisture and the like, and it is possible to heat the object to be heated through a direct contact. In addition, it is possible to coat the ceramic heating element with a catalyst such as a metal or a metal oxide.

Since the activity of the coated catalyst is maximized, when heating the heating element, over a predetermined temperature, a specific component can be easily decomposed. Therefore, it is possible not only to heat a liquid passing through a plurality of paths but also to decompose and remove a specific component by using the catalysis.

Therefore, the ceramic heating element, which is manufactured by using the ceramic heating element composition according to the present invention, can be applied to an odor removing machine in the conventional industry and in the agriculture and stockbreeding field, a ventilation apparatus, and a machine for removing components which are banned according to an environmental law.

Although the present invention has been described with respect to the embodiments illustrated in the figures, it is to be appreciated that these are provided for merely examplary purposes and a person skilled in the art can derive various modifications and equivalent embodiments from them. Therefore, the genuine protection scope of the present invention should only be defined by the appended claims.

### INDUSTRIAL APPLICABILITY

The present invention can be applied in various fields requiring heating such as a food dryer, a hair dryer, an instant water heater, a hot and cold water dispenser, an animal washing and drying machine, an odor removing machine in the conventional industry and in the agriculture and stockbreeding field, a ventilation apparatus, a removing machine for components which are banned according to an environmental law and the like.

## Claims

1. A porous ceramic heating element composition comprising:
a mixture including a ceramic mixed powder, which is formed by mixing at least one of an aluminum nitride and a silicon nitride with a silicon carbide powder, and a silicon-based metal powder which is mixed with the ceramic mixed powder;
a pore agent, wherein 0.5 parts by weight to 5 parts by weight of the pore agent is mixed with 100 parts by weight of the mixture; and
a binder which is mixed with the mixture and maintains the bonding between the ceramic mixed powder and the metal powder, wherein 20 parts by weight to 30 parts by weight of the binder is included in 100 parts by weight of the mixture.

2. The porous ceramic heating element composition according to claim 1, wherein the ceramic mixed powder included in the mixture amounts to 80 wt% to 95 wt% of the overall weight of the mixture.

3. The porous ceramic heating element composition according to claim 1, wherein a ratio of the aluminum nitride or the silicon nitride with respect to the silicon carbide powder amounts to 0.1 parts by weight to 2 parts by weight in 100 parts by weight of the silicon carbide powder.

4. The porous ceramic heating element composition according to claim 1, wherein the metal powder further includes at least one element selected from an aluminum, a copper, and a nickel.

5. The porous ceramic heating element composition according to claim 1, wherein the pore agent is a carbon black or a graphite with a particle size of 0.1 *µ*m to 5 *µ*m.

6. The porous ceramic heating element composition according to claim 1, wherein the binder belongs to a methyl cellulose family.

7. A ceramic heating structure manufactured through molding by using:
a mixture including a ceramic mixed powder, which is formed by mixing at least one of an aluminum nitride and a silicon nitride with a silicon carbide powder, and a silicon-based metal powder which is mixed with the ceramic mixed powder;
a pore agent, wherein 0.5 parts by weight to 5 parts by weight of the pore agent is mixed with 100 parts by weight of the mixture; and
a binder which is mixed with the mixture and maintains the bonding between the ceramic mixed powder and the metal powder, wherein 20 parts by weight to 30 parts by weight of the binder is included in 100 parts by weight of the mixture,
wherein the ceramic heating structure comprises a plurality of air paths through which the air to be heated passes.

8. The ceramic heating structure according to claim 7 wherein the heating structure includes pores for adsorbing and removing by pyrolysis a pollution substance in the air passing through the air path, wherein the pores are formed by removing the pore agent by the heat applied while manufacturing the ceramic heating structure.
